(19) Europäisches Patentamt

European Patent Office

Office européen des brevets

(11) **EP 0 847 748 A2**

(12) **EUROPÄISCHE PATENTANMELDUNG**

(43) Veröffentlichungstag:
17.06.1998 Patentblatt 1998/25

(51) Int. Cl.$^6$: **A61K 7/06**

(21) Anmeldenummer: 97121028.1

(22) Anmeldetag: 29.11.1997

(84) Benannte Vertragsstaaten:
AT BE CH DE DK ES FI FR GB GR IE IT LI LU MC NL PT SE
Benannte Erstreckungsstaaten:
AL LT LV MK RO SI

(30) Priorität: 14.12.1996 DE 19652154

(71) Anmelder:
**Wella Aktiengesellschaft**
**64274 Darmstadt (DE)**

(72) Erfinder:
• **Schmenger, Jürgen**
**64331 Weiterstadt (DE)**
• **Wendel, Harald**
**64372 Ober-Ramstadt (DE)**

(54) **Silikonhaltiges Gel zur Haarbehandlung**

(57) Gegenstand der vorliegenden Erfindung ist ein Gel zur Haarbehandlung mit einem Gehalt an

(A) 0,1 bis 49 Gewichtsprozent mindestens eines Copolymeren aus Dimethylsiloxan und Polyoxyalkylen,
(B) 0,1 bis 90 Gewichtsprozent mindestens einer wasserunlöslichen, hydrophoben Silikonverbindung und
(C) 0,1 bis 49 Gewichtsprozent Wasser,

wobei das Verhältnis von Komponente (A) zu Komponente (C) von 0,9 : 1 bis 1,1 : 1 beträgt.
Die Gele sind zur Verwendung in Haarglanzmitteln geeignet. Besonders klare Gele werden erhalten, wenn das Mischungsverhältnis der Komponenten (A) und (C) etwa bei 1:1 liegt.

EP 0 847 748 A2

**Beschreibung**

Die Erfindung betrifft ein Gel zur Haarbehandlung mit einem Gehalt an mindestens einem Copolymeren aus Dimethylsiloxan und Polyoxyalkylen, insbesondere Polyoxyethylen und/oder Polyoxypropylen, mindestens einer wasserunlöslichen, hydrophoben Silikonverbindung und Wasser.

Haarbehandlungsmittel in Form einer hochviskosen, gelartigen Mischung sind nach dem Stand der Technik hinreichend bekannt. Zum einen sind solche gelartigen Mischungen als sogenannte Mikroemulsionen bekannt, die hauptsächlich aus Paraffinölen, ethoxylierten Fettalkoholen (z. B. Oleylcetylalkohol, ethoxyliert mit ca. 5 Mol Ethylenoxid) und Wasser bestehen. Die bekannten Mikroemulsionen enthalten keine Silikone und erreichen ihre Gelstruktur in einem ganz bestimmten Mischungsverhältnis von Paraffinöl, ethoxyliertem Fettalkohol und Wasser. Der Nachteil dieser gelartigen Mikroemulsionen liegt in ihrem umständlichen Herstellungsverfahren. Mikroemulsionen müssen bei höheren Temperaturen gerührt werden und unterliegen einer empfindlichen Abkühlphase, in der sich die Gelstruktur ausbildet.

Zum anderen sind gelartige Mischungen aus reinen Silikonen bekannt, die hauptsächlich aus Cyclomethiconen, Dimethiconen oder deren Gemischen bestehen. Diese Silikonmischungen enthalten aber kein Wasser und bilden nur in wasserfreien Formulierungen ein klares Gel. Die wasserfreien Silikongele weisen den Nachteil auf, daß sie nur unzureichend auswaschbar und daher schwer wieder vom Haar entfernbar sind.

Es bestand daher die Aufgabe, ein Gel zur Verfügung zu stellen, welches als Haarglanzmittel verwendbar, einfach herzustellen und gut auswaschbar ist.

Es wurde nun gefunden, daß ein Gel mit einem Gehalt an einer Kombination von wasserunlöslichen Polysiloxanen oder Polysiloxanderivaten, wasserlöslichen Polysiloxanderivaten und Wasser die gestellte Aufgabe löst. Es werden im Kaltverfahren herstellbare, klare, hochviskose, Gele erhalten, die hervorragend dazu geeignet sind, in Haarglanzmitteln eingesetzt zu werden.

Gegenstand der vorliegenden Erfindung ist daher ein Gel zur Haarbehandlung mit einem Gehalt an

(A) 0,1 bis 49 Gewichtsprozent, vorzugsweise 1 bis 40 Gewichtsprozent mindestens eines Copolymeren aus Dimethylsiloxan und Polyoxyalkylen, vorzugsweise Polyoxyethylen und /oder Polyoxypropylen,
(B) 0,1 bis 90 Gewichtsprozent, vorzugsweise 1 bis 70 Gewichtsprozent mindestens einer wasserunlöslichen, hydrophoben Silikonverbindung und
(C) 0,1 bis 49 Gewichtsprozent, vorzugsweise 1 bis 40 Gewichtsprozent, besonders bevorzugt 5 bis 40 Gewichtsprozent Wasser.

Der Trübungsgrad der Gele kann durch die Variation des Mischungsverhältnisses eingestellt werden. Besonders klare Gele lassen sich herstellen, wenn das Mischungsverhältnis der Komponenten (A) und (C) etwa 1:1, das heißt 0,9 : 1 bis 1,1 : 1, vorzugsweise 0,95 : 1 bis 1,05 : 1 beträgt. Die Gele sind umso klarer, je näher das Mischungsverhältnis der Komponenten (A) und (C) bei 1 : 1 liegt. Bei Abweichungen von diesem Mischungsverhältnis werden opake und eingetrübte Gele erhalten.

Die als Komponente (A) geeigneten Polymere sind Copolymere mit Polydimethylsiloxaneinheiten und mit hochwasserlöslichen Polyoxyalkylengruppen, insbesondere mit Polyoxyethylen- und/oder Polyoxypropylengrupppen und werden auch als Dimethylsiloxanglykolcopolymere oder als Dimethicon Copolyole bezeichnet. Geeignet sind beispielsweise Dimethicon Copolyole der Formel

$$Me_3Si-O-[SiMeR^1-O-]_m-[SiMeR^2-O]_n-SiMe_3$$

wobei $R^1$ eine C1-C18-Alkylgruppe, $R^2$ die Gruppe -$(CH2)_y$-O-(A-O)$_z$-X, A eine Ethylen- oder Propylengruppe, X Wasserstoff oder eine C1-C4-Alkylgruppe, n, m und z Zahlen von 2 bis 25 und y eine Zahl zwischen 1 und 10 bedeuten.

Als Komponente (B) geeignete Silikonverbindungen sind unter anderem Polyalkylsiloxane, insbesondere Polydimethylsiloxan (Dimethicon), Polyarylsiloxane, Polyalkylarylsiloxane, Polyalkylaminosiloxane, α-Hydro-ω-hydroxypolyoxydimethylsilylen (Dimethiconol), cyclisches Dimethylsiloxan (Cyclomethicon), Trimethyl(octadecyloxy)silan (Stearoxytrimethylsilan). Die in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind. Bevorzugte Silikone sind Dimethicon, Cyclomethicon und Dimethiconol. Auch Mischungen von Silikonpolymeren sind geeignet wie zum Beispiel eine Mischung aus Dimethicon und Dimethiconol.

Beispiele für geeignete lineare Polydimethylsiloxane sind niedrigviskose Polydimethylsiloxane mit einer Viskosität von 0,1 bis 20.000 mm$^2$ s$^{-1}$, vorzugsweise mit einer Viskosität von 0,5 bis 500 mm$^2$ s$^{-1}$. Beispiele für geeignete cyclische Polydimethylsiloxane sind solche mit 4 bis 6 Siliziumatomen oder deren Gemische. Besonders geeignet ist auch ein Gemisch aus cyclischen und linearen Polydimethylsiloxanen.

In einer weiteren Ausführungsform enthält das erfindungsgemäße Gel zusätzlich als Komponente (D) mindestens ein filmbildendes und haarfestigendes Polymer. Dieses zusätzliche Polymer kann ein synthetisches oder ein natürliches Polymer sein und nichtionischen, kationischen, anionischen oder amphoteren Charakter haben und wird bevorzugt in einer Menge von 0,01 bis 50 Gewichtsprozent, besonders bevorzugt in einer Menge von 0,5 bis 20 Gewichtsprozent eingesetzt.

Unter filmbildenden und haarfestigenden Polymeren werden solche Polymere verstanden, welche allein in 0,1 bis 5 %iger wäßriger, alkoholischer oder wäßrigalkoholischer Lösung angewandt in der Lage sind, auf dem Haar einen Polymerfilm abzuscheiden und auf diese Weise das Haar zu festigen.

Geeignete synthetische nichtionische, filmbildende haarfestigende Polymere sind zum Beispiel Homopolymere des Vinylpyrrolidons, Homopolymere des N-Vinylformamids und Copolymerisate aus Vinylpyrrolidon und Vinylacetat.

Geeignete synthetische filmbildende, anionische Polymere sind zum Beispiel Crotonsäure-Vinylacetat-Copolymere oder Terpolymere aus Acrylsäure, Ethylacrylat und N-t-Butylacrylamid.

Des weiteren können verschiedene Saccharidtypen verwendet werden, wie zum Beispiel Polysaccharide oder Gemische aus Oligo-, Mono- und Disacchariden. Weitere geeignete, natürliche Polymere sind Cellulosederivate, zum Beispiel Hydroxypropylcellulose mit einem Molekulargewicht von 30.000 bis 50.000 g/mol.

Geeignete amphotere Polymere sind zum Beispiel Copolymere aus Octylacrylamid, t-Butylaminoethylmethacrylat und zwei oder mehr Comonomeren, ausgewählt aus Acrylsäure, Methacrylsäure oder deren Estern.

Von den kationischen Polymeren, die in dem erfindungsgemäßen Mittel enthalten sein können, sind zum Beispiel Polyvinylpyrrolidon/Dimethylaminoethylmethacrylat-Copolymer, das Copolymer aus Polyvinylpyrrolidon und Imidazoliminmethochlorid, das Terpolymer aus Dimethyldiallylammoniumchorid, Natriumacrylat und Acrylamid, das Terpolymer aus Vinylpyrrolidon, Dimethylaminoethylmethacrylat und Vinylcaprolactam, quaternierte Ammoniumsalze der Hydroxyethylcellulose, das Vinylpyrrolidon/Methacrylamidopropyltrimethylammoniumchlorid Copolymer und diquaternäre Polydimethylsiloxane.

Des weiteren können in dem erfindungsgemäßen Mittel Polymere mit verdickender Wirkung eingesetzt werden. Von den Verdickern, die in dem erfindungsgemäßen Mittel enthalten sein können, sind Homopolymere der Acrylsäure mit einem Molekulargewicht von 2.000.000 bis 6.000.000 g/mol zu nennen. Als weitere Verdicker kann das erfindungsgemäße Mittel ein Acrylsäurehomopolymer mit einem Molekulargewicht von 4.000.000 g/mol enthalten. Weitere Verdicker sind beispielsweise das Polymer aus Acrylsäure und Acrylamid (Natriumsalz) mit einem Molekulargewicht von 2.000.000 bis 6.000.000 g/mol.

Das erfindungsgemäße Mittel wird bevorzugt in einem wäßrigen Milieu konfektioniert, aber es sind auch wäßrig/alkoholische Rezepturen möglich. Des weiteren können Lösungsmittel oder ein Gemisch aus Lösungsmitteln mit einem Siedepunkt unter 400 °C in einer Menge von 0,01 bis 20 Gewichtsprozent, bevorzugt von 0,1 bis 10 Gewichtsprozent eingesetzt werden.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Selbstverständlich können die erfindungsgemäßen Mittel auch weitere übliche kosmetische Zusätze wie beispielsweise nichthaarfestigende nichtionische Polymere, wie zum Beispiel Polyethylenglykol mit einem Molekulargewicht von 600 g/mol, nichthaarfestigende anionische Polymere und nichthaarfestigende natürliche Polymere sowie deren Kombination in einer Menge von vorzugsweise 0,01 bis 50 Gewichtsprozent; Parfümöle in einer Menge von vorzugsweise 0,01 bis 5 Gewichtsprozent; Netzmittel oder Emulgatoren aus den Klassen der anionischen, kationischen, amphoteren oder nichtionogenen oberflächenaktiven Substanzen wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 bis 30 Gewichtsprozent; ferner Feuchthaltemittel, Farbstoffe, Lichtschutzmittel, Antioxidantien, Glanzgeber und Konservierungsstoffe in einer Menge von vorzugsweise 0,01 bis 10 Gewichtsprozent enthalten.

Das erfindungsgemäße Gel verleiht den damit behandelten Haaren einen schönen Glanz. Das Gel ist aus dem Haar gut auswaschbar.

Die nachfolgenden Beispiele sollen die Erfindung näher erläutern.

Beispiel 1: Hochviskoses Glanzgel

| | |
|---|---|
| 32,0 g | Dimethylsiloxan/Polyoxyethylen Copolymer (GE Silicone SF 1188A der Firma General Electric/USA) |
| 32,0 g | Gemisch aus 15 % Polydimethylsiloxan und 85 % Decamethylcyclopentasiloxan (GE Silicone SF 1214 der Firma General Electric/USA) |
| 0,8 g | Parfümöl |
| 0,2 g | Konservierungsmittel |
| 35,0 g | Wasser, vollentsalzt |
| 100,0 g | |

Das Mittel wird in Form einer hochviskosen, gelartigen Mischung auf das Haar mit den Händen aufgetragen.

Beispiel 2: Glanzgel mit Festigung

| | |
|---|---|
| 20,0 g | Dimethylsiloxan/Polyoxyethylen Copolymer (GE Silicone SF 1188A der Firma General Electric/USA) |
| 0,5 g | Polyvinylpyrrolidon |
| 59,0 g | Octamethylcyclotetrasiloxan |
| 0,1 g | Konservierungsmittel |
| 0,4 g | Parfümöl |

20,0 g    Wasser, vollentsalzt
100,0 g

Das Gel stellt eine hochviskose, klare Masse dar.

Beispiel 3: Parfüm Glanzgel

27,5 g    Dimethylsiloxan/Polyoxyethylen Copolymer (Abil 8842 der Firma Goldschmidt/Deutschland)
40,0 g    Gemisch aus 15 % Polydimethylsiloxan und 85 % Decamethylcyclopentasiloxan (GE Silicone SF 1214 der Firma General Electric/USA)
4,9 g    Parfümöl
0,1 g    Konservierungsmittel
27,5 g    Wasser, vollentsalzt
100,0 g

Das Gel stellt eine hochviskose, klare Masse dar.

Beispiel 4: Fön Glanzgel

30,00 g    Dimethylsiloxan/Polyoxyethylen/Polyoxypropylen Copolymer mit einem Oxyethylen:Oxypropylenverhältnis von 40:60 (Abil 8863 der Firma Goldschmidt/Deutschland)
38,00 g    Gemisch aus 15 % Polydimethylsiloxan und 85 % Decamethylcyclopentasiloxan (GE Silicone SF 1214 der Firma General Electric/USA)
0,19 g    Vinylpyrrolidon/Dimethylaminoethylmethacrylat Copolymer
0,10 g    Konservierungsmittel
0,50 g    Parfümöl
0,40 g    hydriertes Rizinusöl
30,81 g    Wasser, vollentsalzt
100,00 g

Das Gel stellt eine hochviskose, klare Masse dar.

Beispiel 5: Glanzgel mit Perlglanzeffekt

32,0 g    Dimethylsiloxan/Polyoxyethylen Copolymer (GE Silicone SF 1188A der Firma General Electric/USA)
32,0 g    Gemisch aus 15 % Polydimethylsiloxan und 85 % Decamethylcyclopentasiloxan (GE Silicone SF 1214 der Firma General Electric/USA)
0,5 g    Parfümöl
0,1 g    Konservierungsmittel
0,4 g    Timiron Gleamer Flake (Titandioxid 15%, Mica 85%)
35,0 g    Wasser, vollentsalzt
100,0 g

Das Gel stellt eine hochviskose, klare Masse dar.

Beispiel 6: Fön Glanzlotion

32,0 g    Dimethylsiloxan/Polyoxyethylen Copolymer (DMC 6031 der Firma Wacker/Deutschland)
35,0 g    Octamethylcyclotetrasiloxan
0,2 g    Polydimethyldiallylammoniumchlorid
0,4 g    Parfümöl
0,1 g    Konservierungsmittel
32,3 g    Wasser, vollentsalzt
100,0 g

Das Mittel liegt in Form einer dickflüssigen Lotion vor.

Beispiel 7

40,0 g    Dimethylsiloxan/Polyoxyethylen Copolymer (GE Silicone SF 1188A der Firma General Electric/USA)
19,0 g    Gemisch aus 15 % Polydimethylsiloxan und 85 % Decamethylcyclopentasiloxan (GE Silicone SF 1214 der Firma General Electric/USA)
1,0 g    Gemisch aus 87% Dimethicone und 13% Dimethiconol (Dow Corning 1403 der Firma Dow Corning, USA)
40,0 g    Wasser, vollentsalzt
100,0 g

Das Mittel liegt in Form eines hochviskosen, klaren Gels vor.

Beispiel 8

40,0 g    Dimethylsiloxan/Polyoxyethylen Copolymer (GE Silicone SF 1188A der Firma General Electric/USA)
20,0 g    Gemisch aus 87% Dimethicone und 13% Dimethiconol (Dow Corning 1403 der Firma Dow Corning, USA)
40,0 g    Wasser, vollentsalzt
100,0 g

Das Mittel liegt in Form eines hochviskosen, klaren Gels vor.

Beispiel 9

40,0 g    Dimethylsiloxan/Polyoxyethylen Copolymer (GE Silicone SF 1188A der Firma General Electric/USA)
20,0 g    Gemisch aus 87% Dimethicone und 13% Dimethiconol (Dow Corning 1401 der Firma Dow Corning, USA)
40,0 g    Wasser, vollentsalzt
100,0 g

Das Mittel liegt in Form eines hochviskosen, klaren Gels vor.

**Patentansprüche**

1.  Gel zur Haarbehandlung mit einem Gehalt an

    (A) 0,1 bis 49 Gewichtsprozent mindestens eines Copolymeren aus Dimethylsiloxan und Polyoxyalkylen,
    (B) 0,1 bis 90 Gewichtsprozent mindestens einer wasserunlöslichen, hydrophoben Silikonverbindung und
    (C) 0,1 bis 49 Gewichtsprozent Wasser,

    wobei das Verhältnis von Komponente (A) zu Komponente (C) von 0,9 : 1 bis 1,1 : 1 beträgt.

2.  Gel nach Anspruch 1, dadurch gekennzeichnet, daß es als Komponente (A) mindestens ein Copolymer aus Dimethylsiloxan und Polyoxyethylen und/oder Polyoxypropylen enthält.

3.  Gel nach einem der vorstehenden Ansprüche, dadurch gekennzeichnet, daß das wasserunlösliche Silikonderivat ausgewählt ist aus cyclischen oder linearen Polydimethylsiloxanen und Polydimethylsiloxanen mit Hydroxylendgruppen.

4.  Gel nach einem der vorstehenden Ansprüche, dadurch gekennzeichnet, daß es zusätzlich

    (D) 0,01 bis 50 Gewichtsprozent mindestens eines filmbildenden und haarfestigenden Polymers enthält.